(19)

Europäisches
Patentamt

European
Patent Office

Office européen
des brevets

(11) **EP 4 068 298 A1**

(12) **EUROPEAN PATENT APPLICATION**

(43) Date of publication:
**05.10.2022 Bulletin 2022/40**

(21) Application number: **21166235.8**

(22) Date of filing: **31.03.2021**

(51) International Patent Classification (IPC):
***G16H 20/70*** *(2018.01)*

(52) Cooperative Patent Classification (CPC):
**G16H 10/20; G16H 20/70**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Sonova AG
8712 Stäfa (CH)**

(72) Inventors:
- **Haller, Markus
  2020 BEIRUT (LB)**
- **Hauptmann, Christian
  82319 Starnberg (DE)**
- **Hauptmann, Nicole
  82319 Starnberg (DE)**

(74) Representative: **Eisenführ Speiser
Patentanwälte Rechtsanwälte PartGmbB
Postfach 31 02 60
80102 München (DE)**

(54) **COGNITIVE BEHAVIORAL THERAPY (CBT) METHOD, SYSTEM AND APPLICATION FOR MANAGING TINNITUS**

(57) The present invention relates primarily to a tinnitus management system and a computer-implemented method for managing tinnitus. The system comprises a survey developing unit, a progress determining unit, a performance evaluation unit, and a feedback unit. The survey developing unit develops and/or adapts, after a first training section, a subsequent, second training section or a following part of a survey based on a performance indicator determined for the first training section.

**EP 4 068 298 A1**

**Description**

The present invention resides in the field of tinnitus management and specifically concerns a cognitive behavioral therapy (CBT) method, system and application for managing tinnitus.

Cognitive behavioral therapy (CBT) is a psycho-social intervention that aims to improve mental health. CBT focuses on challenging and changing unhelpful cognitive distortions (e.g. thoughts, beliefs, and attitudes) and behaviors, improving emotional regulation, and the development of personal coping strategies that target solving current problems. Originally, it was designed to treat depression, but its uses have been expanded to include treatment of a number of mental health conditions, including tinnitus.

Tinnitus is defined as auditory perception without external sound (phantom noise). Tinnitus affects about 10 to 15% of the population. The phantom noise may vary in pitch from a low roar to a high squeal, and it may be heard in one or both ears. In some cases, the sound can be so loud it can interfere with the ability to concentrate or hear external sound. Tinnitus may be present all the time, or it may come and go. Subjective tinnitus is tinnitus that only the affected subject can hear. This is the most common type of tinnitus.

It is important to understand that tinnitus is not an actual disease but a change in how someone perceives the internal workings of the hearing system. The sound that is perceived can vary from person to person, but is most likely to be a high-pitched noise. The perception of these high-pitched noises is most likely caused by a degree of overactivity within the nerve and brain systems related to hearing function. Tinnitus can be triggered by other health conditions like hearing loss, loud noise or stress.

Tinnitus can significantly affect quality of life. Although it affects people differently, an affected subject may also experience fatigue, stress, sleep problems, trouble concentrating, memory problems, depression, anxiety and irritability. There is currently no cure for tinnitus.

CBT addresses the affected subject's reaction to tinnitus. It aims not to eliminate auditory perception as sound but to reduce or correct the subject's negative response thereto. In this way, subjects suffering from tinnitus (herein referred in short to as the subject) can function well despite the presence of tinnitus. CBT is based on the belief that thought distortions and maladaptive behaviors play a role in the development and maintenance of tinnitus and that symptoms and associated distress can be reduced by teaching new information-processing skills and coping mechanisms. Studies have supported the efficacy of CBT for managing tinnitus.

Because CBT is a "problem-focused" and "action-oriented" form of therapy, the therapist's role is essential in traditional CBT therapy. The therapist's role is to assist the subject in finding and practicing effective strategies to address the identified goals and decrease symptoms of the disorder (tinnitus). A traditional CBT program consists of face-to-face sessions between the suffering subject and therapist, made up of 6 to 18 sessions of around an hour each with a gap of 1 to 3 weeks between sessions. This initial program might be followed by some booster sessions, for instance after one month and three months. CBT has also been found to be effective if subject and therapist type in real time to each other over computer links. Still, effective cognitive behavioral therapy is dependent on a therapeutic alliance between the therapist and the subject.

A major drawback of the traditional CBT therapy is that the preparations before and after each session can be more or less laborious and time-consuming for the subject. Appointments need to be made. The subject has to commute between home and the therapist's office, and might to wait in a waiting room. In addition, sessions are traditionally planned in fixed, typically weekly, time intervals. The intervals not only places a heavy burden on the subject to come the weekly face-to-face therapies but also do not necessarily meet the subject's need. Finally, there is a high risk that the patient is lost in the time between the sessions.

The problem to be solved by the present invention is to simplify the complex management of subjective tinnitus. In particular, it was an aim to provide a less tedious and less time-consuming approach that is further adaptable to a subject's actual need. This problem is solved by a tinnitus management system as defined in appended claim 1. The tinnitus management system comprises:

> a survey developing unit for developing and transmitting a survey (also referred to herein as CBT program) to a user communication unit, the survey including a plurality of training sections, each training section including a plurality of training segments to be completed within a respective target timeframe, the training segments including educational elements and/or practical exercises,
> a progress determining unit for receiving and evaluating a user response received from the user communication unit, and repeatedly determining the number of completed training segments and/or training sections,
> a performance evaluation unit for comparing the target timeframe received from the survey developing unit with the actual duration needed for completing the respective training section and for determining a performance indicator indicative for the relative speed of completing the respective training section, and
> a feedback unit for selecting one of a plurality of feedbacks based on the performance indicator received from the performance evaluation unit and for transmitting, after each training section, the feedback to the user communication unit.

Moreover, according to the present invention, the survey developing unit develops and/or adapts, after a (first) training section, the subsequent (second) training section based on the performance indicator determined for the (first) training section. Preferably, the developing and/or adaptation is carried out after each training section.

The phrase "managing tinnitus" as used herein means managing reactions to tinnitus. The sound of tinnitus cannot be changed unless tinnitus is a symptom of another condition that is treated. However, treating such other condition is not within the focus of the present invention. Accordingly, managing tinnitus is related to how a tinnitus sufferer (herein also referred to as user) reacts to the sound. The phrase "managing tinnitus" includes, without limitation, any measure that assists a patient to perceive the patient's tinnitus less bad by positively influencing the patient's (subjective) attitude towards the tinnitus. The present invention provides means and methods that assist a user to experience tinnitus, in particular, subjective tinnitus, less burdensome.

The term "repeatedly" denotes an action (herein: determining the user's progress in the user survey) that is performed at least twice. Preferably, the action is coupled to another event and is performed at least as often as the event occurs. Herein, it is envisaged that determining the user survey progress is coupled to the completion of the training segment and/or training section. Thus, the determining may be performed upon or after completion of a (e.g., each) training segment and/or a (e.g., each) training section. In this way, the system always "knows" the user's progress, i.e. how many training sections and/or training segments a user has already completed.

In various embodiments, the tinnitus management system corresponds to or is part of a computer network providing a plurality of user access to one or more applications. An application may include one or more modules/functions. The computer network includes at least one host computers and a plurality of terminals at which respective users may request applications.

The tinnitus survey developing unit manages one or more surveys (treatment plans) stored in the computer network, for instance, on the at least one host computer. The one or more surveys may include generic contents, that is to say predetermined contents to be finished by every user. Examples in this regard include educational contents about different topics, for instance, what tinnitus is and how it develops, about factors influencing tinnitus and its subjective perception as more or less burden-some, etc. For the internalization of the educational content, it is preferred that the content includes a questionnaire regarding what has been taught by the respective content. Further examples of contents include practical exercises in the form of performing a hearing test, hearing to a (virtual) therapist, performing relaxation exercises, experiencing sound therapy, etc. Optionally, the survey includes diagnostic elements for determining, or acquiring results with respect to, the severity of the subject's tinnitus (e.g., using clinically standardized measures like Tinnitus Handicap Inventory (THI), Tinnitus Functional Index (TFI), Generalized Anxiety Disorder Scale-7 (GAD-7) and/or Patient Health Questionnaire Scale 9 (PHQ-9) questionnaire). At least a part of the content is related to CBT. Moreover, the one or more surveys may include user-specific contents that are specifically adapted to the user. For instance, some of the previous exemplary contents can occur only for specific users. Whether the survey includes generic and/or specific contents, it is an important aspect of the present invention that the survey adapts with the progress of the user during completion of the survey so that each user finds his/her individual optimal pace. Not only the pace but also the sequence of the contents may be adapted. For instance, if a questionnaire reveals (e.g. based on the number of correct answers) that a subject has not yet sufficiently memorized a particular content, the tinnitus survey developing unit adapts the survey so that the user will have to repeat the particular content. Therefore, the survey developing unit repeatedly communicates with the user communication unit.

Contents preferably relate to education and counseling or CBT. Specific examples of contents include, without limitation:

- educating the user, for instance, about the different types of tinnitus and the different potential causes of tinnitus. The user may get information about available treatment options and receives some hints how to better deal with his/her tinnitus to support habituation,

- tracking the patient progress by questions,

- training in relaxation exercises to reduce stress, which help to reduce tinnitus burden,

- relaxation trainings may also include trainings where patient is confronted with his tinnitus,

- training in directing the patient's attention and paying attention to all his/her senses,

- introducing the ABC model and vicious spiral,

- training to use questioning methods to identify irrational thoughts,

- training to identify and escape thinking traps,

- training in using counter and coping strategies,

- introducing the salutogenesis model,

- tracking of the user's progress by questions,

- tracking the user's compliance,

The survey is divided into training sections. A training section can be viewed as a concluded training session for/after which the performance of the user is determined. Each training section may be completable within several days such as 3, 4, 5, 6, 7, 8, 9, 10, 11, 12, 13, 14, 15, 16, 17, 18, 19, 20 or more days, preferably within 5 to 14 days. A training section can be further divided into training segments.

Each training segment preferably includes only one content, for instance, one topic or one relaxation exercise. Moreover, each training segment may be completable within one day, e.g., within 5 minutes and 120 minutes, preferably within 10 minutes and 40 minutes. Shorter trainings are less effective and longer trainings are tedious. Each training segment is to be completed within a target duration. The target duration is preferably defined in days, meaning that the target duration specifies on which day (or after how many days after start of the respective training section) a training segment should be finished.

The progress determining unit tracks the progress of the user in completing the survey. To this end, the progress determining unit, repeatedly (e.g., after each training segment) evaluates whether the user has performed the respective training segment. It is further preferred that the progress determining unit evaluates the feedback to the respective training segment provided by the user, for instance, whether the user has given the correct answers. Thereby, the progress determining unit gathers information on the number of completed training segments and, hence, training sections.

The performance evaluation unit provides a performance indicator. The indicator is a qualitative or quantitative measure that is indicative for the speed of the user. In particular, the indicator may indicate whether the respective user has completed all training segments of a training section exactly within the predetermined target timeframe, or whether he/she was faster or slower than that. The performance indicator is preferably determined after each training section.

Based on the performance indicator, the feedback unit provides feedback to the respective user, preferably directly after completion of a (preferably each) training section. That means, the feedback immediately appears on the screen of the respective user's terminal after he/she has finished a training section. The feedback is dependent on the performance of the respective user. For example, a slow performer may be supported by an encouraging feedback, whereas decelerating words may take a burden off a fast performer.

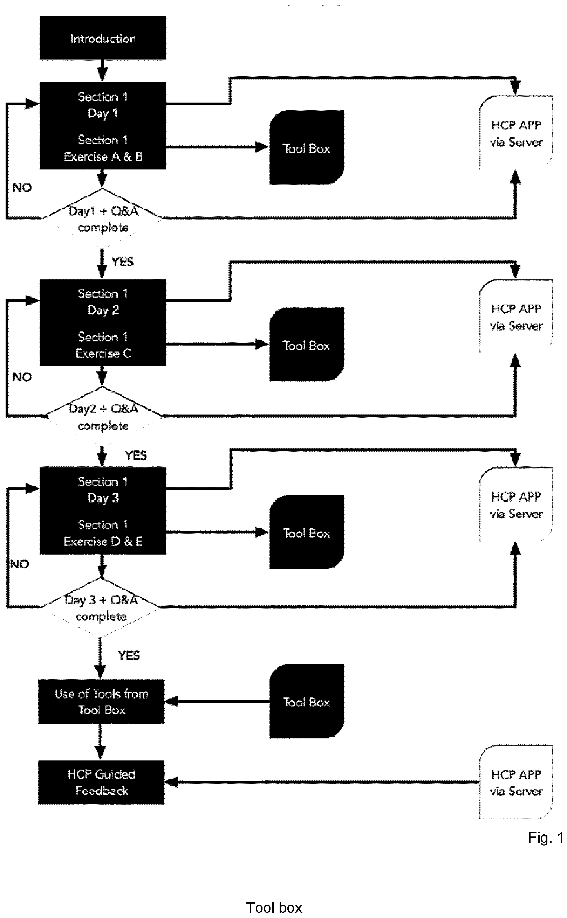

A specific implementation of the feedback functionality is described in the following. According to this implementation, the user is guided by a number of feedback screens, e.g., four feedback screens in the progress of the journey. A first feedback screen is presented after, for instance, seven daily training segments. The initial seven training segments contain, let's say, five practical exercises, which the user should do on a daily basis on top of the daily educational contents. The system tracks, if each daily segment is finished, e.g. by tracking the answers of the questions, but also by tracking, if the content was used (e.g. sound files played). Only if the last daily segment was finished, the next daily segment can be started. On each day only one daily segment can be consumed (see Fig. 1). Therefore, the minimal duration of the seven daily segments is known. But the individual user may conduct the seven daily segments more slowly, and he/she can do more exercises. Both numbers (duration of all seven daily segments [number of days] and number of performed exercises [number]) are known to the system and are used to control the feedback. These numbers are denoted key performance indicators. A target window can be defined for both numbers:

Duration of first seven daily segments: [8, 11]

Number of exercises done: [6, 12]

Based on the individual key performance indicators, nine different situations (SIT) can be defined taking into account the user's compliance with respect to the predetermined duration of the first seven daily segments (CON 1) and the number of exercises done (CON 2).

| SIT A1 | Ambitious user, slow on exercises | CON 1 < Min | CON 2 < Min (less than 6 exercised done) |
|--------|-----------------------------------|-------------|------------------------------------------|
| SIT A2 | Ambitious user | | CON 2 in target |
| SIT A3 | Very Ambitious user | | CON 2 > Max |
| SIT B1 | Good user, but a bit slow on exercises | CON 1 in target | CON 2 < Min |
| SIT B2 | Good user | | CON 2 in target |
| SIT B3 | Good user but doing exercises a lot | | CON 2 > Max |
| SIT C1 | Slow & incompliant user | CON 1 > Max | CON 2 < Min |
| SIT C2 | Slow user | | CON 2 in target |
| SIT C3 | Slow user but doing exercises a lot | | CON 2 > Max |

In the above example, "CON 1 < Min" means that the user completed the first seven daily segments in less than 8 days, "CON 1 in target" means that the user completed the first seven daily segments in between 8 to 11 days, "CON 1 > Max" means that the user completed the first seven daily segments in more than 11 days), "CON 2 < Min" means that the user did less than 6 exercises, "CON 2 in target" means that the user did between 6 and 12 exercises, "CON 2 > Max" means that the user did more than 12 exercises.

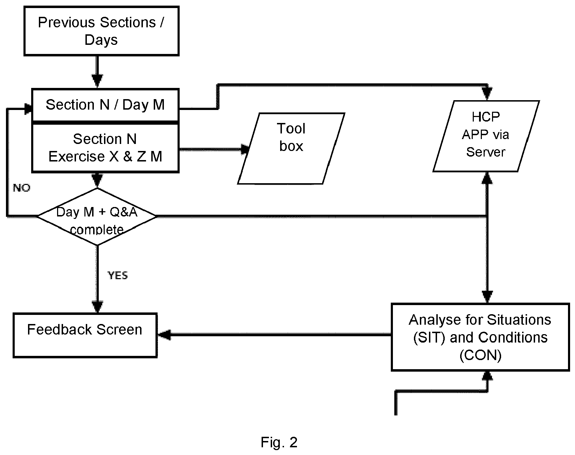

For each such situation, a specific text is defined and used in the feedback screen. E.g. for SIT A3 the feedback contains praising the motivation and progress of the patient, and a proposal that the particular user could progress slightly slower, also allow himself to take a day off. E.g. for SIT C1 the feedback would contain praising the motivation and progress of the user as well, but combined with a reminder, that it is important to keep track of the program and to do the exercises on a daily basis (see Fig. 2). Similarly, for the next 7 daily segments of the journey (CBT program) different target windows are defined, e.g. duration of first seven daily segments: [8,11] and number of exercises done: [3,7] (latter is lower, since no new exercises are part of the daily segments). Again, a similar structure could be used to define the different situations. The text for the second feedback screen could be adapted from the first feedback screen. For the third feedback screen (after 5 further daily segments) new target windows are defined, e.g. duration of first seven daily segments: [6,9] (since only 5 daily segments were between 2nd and 3rd feedback screen) and number of exercises done: [2,5] (latter is lower, since no new exercises are part of the daily segments and only 5 days minimum available).

On the second last daily segment, the user is asked to perform a THI (and GAD-7 and PHQ-9 may be requested initially). This information is compared to the initial values of THI and therapy progress is calculated by calculating the relative change to baseline values, i.e. negative changes indicate improvements of symptoms and positive changes indicate worsening of symptoms. The relative change of outcome measures and the number of exercises done (target window for 4th feedback screen [2,4], since only 4 daily segments contained) is used as basis for the feedback given to the patient. If THI indicates, that symptoms didn't change or worsened slightly during the journey (CBT therapy), patient would get feedback to visit ENT or specialist and might need more individual treatment. If THI indicates, that symptoms are slightly improved, patient gets positive feedback, user is informed, that changing tinnitus symptoms takes longer times, he should continue with the exercises. If THI strongly improves, user gets positive feedback and is also asked to continue with exercises to stabilize therapy success.

Moreover, based on the performance indicator, the survey developing unit adapts and/or develops the subsequent training section. For instance, the survey developing unit defines and/or adapts the number of training segments in the subsequent training section. The survey developing unit may also define and/or adapt the content, for instance, as regards a ratio of the educational contents to the practical exercises. Another possibility is to define and/or adapt the target timeframe of the subsequent training section. As mentioned above, one advantage of the present invention is that the user can define his/her own pace. That means, the target timeframe serves as a guidance but does not force the user to exactly follow the predetermined speed. Deviations therefrom are possible and are taken into account in order to develop or adapt the subsequent training section(s). Thereby, less pressure is put on the user, which is assumed to have a positive effect on the success of the "tinnitus managment".

The system preferably comprises a plurality of user communication units, each one associated with a specific user and implemented on a terminal (as described above) of the respective user. The terminals can be selected from the group consisting of personal computers, mobile computers including tablets and smartphones. The connection between the one or more host computers and the plurality of terminals can be of a direct type, that is to say line-connected, or of an indirect type via a wireless connection. Moreover, the various units described herein such as the tinnitus survey developing unit, user communication unit, progress determining unit, performance evaluation unit, feedback unit, health care professional (HCP) unit and wellbeing evaluation unit as far as present, are communicatively connected with each other.

The HCP unit is an optional element of the system. It allows an HCP to track progress of a user and/or to track performance of a user and/or to communicate with a user. The system may comprise more than one HCP unit, whereby it is preferred that each HCP unit is associated with a different group of users. To track progress and/or performance of a user, the HCP unit communicates with the progress determining unit and/or the performance evaluation unit. For instance, the HCP unit may acquire and/or receive the number of completed training sections and/or segments of the user from the progress determining unit and/or the performance indicator from the performance evaluation unit. The HCP unit is preferably implemented on a terminal of the respective HCP. As described above, the terminals are preferably communicatively connected to the one or more host computers and selected from the group consisting of personal computers, mobile computers including tablets and smartphones.

In an embodiment of the invention, the HCP unit allows the HCP to review and modify the feedback signal. That means, before the feedback signal is transmitted to the user communication unit, it is sent to the HCP unit, where the HCP can read and confirm or revise it.

Moreover, the target timeframe that determines the timeframe until a training section is to be completed is preferably defined so that on average one training segment is to be completed in 1 to 3 days. Accordingly, it is preferred that not more than one training segment is completed on the same day. Still, in certain embodiments, it is envisaged that the user may complete two training segments on the same day provided that an educational content alternates a practical exercise. In particular, it is not desired to have two educational contents on the same day. Thus, it is preferred that access of the user to an educational element is disabled, if the progress determining unit determines that a previous educational element has already been completed on the same day.

In a further embodiment of the invention, access of the user to a training segment is disabled, until the progress determining unit determines that each previous training segment has been completed. It is thereby ensured that the training segments are completed in a predetermined order or that the user cannot skip or suspend undesired contents.

The system may further comprise a wellbeing evaluation unit for determining a wellbeing score based on the user's response to a TFI, THI, GAD-7 and/or PHQ-9 questionnaire. The wellbeing score is a quantitative or qualitative measure indicative of the user's wellbeing, for instance with respect to handicap, anxiety and/or depression. As such, the wellbeing score may represent or be correlated with the respective TFI, THI, GAD-7 and/or PHQ-9 score of the user. The wellbeing of the user is preferably determined at least twice at different progress (for example, at the beginning and the end of the survey, or before and after a training segment or training section) in order to determine the success of training segments, training sections or the complete survey. Thus, according to an embodiment of the invention, the wellbeing evaluation unit determines a first wellbeing score and a second wellbeing score at a more advanced progress, and compares the second wellbeing score with the first wellbeing score to determine a wellbeing development score. The wellbeing development score is a qualitative or quantitative measure that indicates whether and/or how much the patient's wellbeing improved, stagnated or worsened.

If the wellbeing development score indicates that the patient's wellbeing improved, the survey is assumed to be successful. It is thus preferred that in this case, the survey development unit includes at least one additional training section in the survey to maintain and strengthen the positive effects of the survey.

If, on the other hand, the wellbeing development score indicates that the patient's wellbeing stagnated or worsened, the survey is assumed to be not successful. Therefore, the survey is preferably discontinued or adapted. For the purposes above, it is evident that the wellbeing evaluation unit is communicatively connected with the wellbeing evaluation unit.

In preferred embodiments of the invention, the survey development unit applies a dynamic, self-optimizing algorithm when including the additional training section based on the wellbeing development score and/or wherein the survey development unit applies a dynamic, self-optimizing algorithm when developing and/or adapting the subsequent training section or the following part of the survey based on the performance indicator. Thereby, not only the optimal contents can be selected for the individual user but also user compliance can be efficiently managed. The process may be controlled by two algorithms, one for the selection of the optimal contents and one for the management of user compliance. The phrase "optimal contents" may refer to an optimal combination of contents (e.g., cognitive behavioral therapy combined with acoustic therapy), and/or an optimal sequence of contents, e.g., psychotherapeutic therapy. Starting from a static model that may be based on input of tinnitus experts, a dynamic, self-optimizing algorithm may be generated by applying machine learning methods. A preferred algorithm employs reinforcement learning and is further described in the following.

Reinforcement learning is a concept, where an agent (the algorithm) independently learns a strategy to maximize an expected value of rewards received. This concept is preferred in the present case, because on the one hand the observed therapy success (e.g. as determined by the wellbeing score) can be used to calculate a reward for the agent and on the other hand the initial decision path given by the tinnitus experts lets the agent already start from a good position and thus a fast convergence can be expected.

In the method of reinforcement learning, at the time of *t* a set of states is assumed $s_t \in S$ (the set of all information about the individual patient). The agent now chooses an action $a_t$ from a set of available actions $a_t \in A$ (the therapy/content offer), and reaches a state $s_{t+1} \in S$ and receives a reward $r_{t+1} \in \mathbb{R}$. The agent's goal is to maximize the sum of the expected reward, where the total reward is discounted by a factor of $\gamma$ is applied:

$$R_t = \sum_{i=0}^{T} \gamma^i \cdot r_{t+i+1} \text{ with } 0 \le \gamma \le 1$$

The discount factor determines the range of the strategy; for $\gamma = 0$ only the outcome of the next step (or the next therapy unit) is considered, whereas for $\gamma = 1$ the results of all following steps (or the complete sequence of therapy units) are taken into account.

In order to maximize the expected profit, and thus also the expected therapeutic success of the individual user, the agent constantly adapts its policy. $\pi : S \rightarrow A$ constantly. The starting point of learning should be a non-deterministic strategy that, based on the experts' suggestions, assigns a certain probability to an action (selected therapy) depending on the initial condition $\pi(s,a) = p(a|s)$. Starting from the initial algorithm of the experts (e.g., an extensive table connecting state space and action space), the fields of the table are updated depending on the reward received and thus the algorithm is modified and optimized, for this purpose, for example, the Temporal Difference Learning method can be used.

Since the wellbeing score and the performance indicator are determined at regular intervals, the course of therapy can be recorded closely. Hence, a so-called bootstrapping can be applied. A simple algorithm may use the following formula:

$$V(s_t) \leftarrow (1 - \alpha)V(s_t) + \alpha(r_{t+1} + \gamma V(s_{t+1})),$$

where a low learning rate $\alpha$ in the case of a changing environment leads to the expectation of a more stable behavior of the algorithm. This algorithm can be complemented by the so-called. $\varepsilon$-greedy policy, where with a probability of $\varepsilon$ a random action (within the expert constraints) is chosen by the agent instead of the most promising action (according to the evaluation function). This makes it possible to find solutions that are not obvious but have high relevance for research. Another useful addition is the so-called reward shaping, where additional rewards are introduced to achieve a better performance of the algorithm.

In addition, or alternatively, to the algorithm for content (therapy) selection and control, an algorithm for compliance management can be implemented. The reward of this agent may be defined as an improved compliance of the patient after an appropriate action. The reward can be calculated from the performance indicator or the corresponding user data. For example, compliance can be easily collected and quantified by the duration of use and completeness of questionnaires. As actions, the agent is provided with a range of actions, including reminders, targeted explanations, encouragement, and reports of therapy effects already achieved. Good compliance is a major contributor to successful therapy, so the overall goal of successful therapy may be furthered by the compliance management. Also in this regard, the method of reinforcement learning is preferred over the other methods of machine learning (e.g. Association Rule Learning, Artificial Neural Networks, Deep Learning, Inductive Logic Programming, Support Vector Machines, Clustering, Bayesian Networks, Representation Learning or Similarity and Metric Learning).

A second aspect of the present invention pertains to a computer-implemented method of managing tinnitus, whereby

a survey developing unit develops and transmits a survey to a user communication unit, the survey including a plurality of training sections, each training section including a plurality of training segments to be completed within a respective target timeframe, the training segments including educational elements and/or practical exercises;

a progress determining unit receives and evaluates a user response received from the user communication unit, and repeatedly determines the number of completed training segments and/or training sections,

a performance evaluation unit compares the target timeframe received from the survey developing unit with the actual duration needed for completing the respective training section and determines a performance indicator indicative for the relative speed of completing the respective training section,

a feedback unit selects one of a plurality of feedbacks based on the performance indicator received from the performance evaluation unit and transmits the feedback, preferably after each training section, to the user communication unit,

the survey developing unit develops and/or adapts, preferably after a (first) training section, the subsequent (second) training section(s) or the following part of the survey based on the performance indicator determined for the immediately preceding (first) training section. Preferably, the developing and/or adaptation is carried out after each training section.

The various units used in the method of the invention, including, for instance the survey developing unit, the user communication unit, the progress determining unit, the performance evaluation unit and the feedback unit, are preferably part of a system as described in the context of the first aspect of the present invention. With respect to further features and embodiments of the method, reference is thus made to the description relating to the system of the first aspect of the invention, which shall be understood to describe corresponding features and embodiments of the method according to the second aspect of the invention.

According to a third aspect of the present invention, the use of a system as described herein for managing tinnitus is provided. As mentioned above, the managing tinnitus is based on/achieved by assisting a user to perceive the tinnitus less burdensome. Whether a tinnitus is perceived as less burdensome can, for instance, be determined based on the THI, TFI, GAD-7 and/or PHQ-9 score(s). Preferably one or more of the THI, TFI, GAD-7 and/or PHQ-9 score(s) are determined before start of the survey or within the survey, whereas the respective score(s) are re-determined at an advanced progress of the survey, in particular at the end of the survey. As regards further features and embodiments of the use, it is referred to the description of the first aspect of the invention, which is meant to provide corresponding features and embodiments of the use according to the third aspect of the invention.

Further aspects, embodiments and advantages of the present invention become apparent from the following examples, the figures and claims, which follow after the brief description of the drawings.

In the drawings:

Figs. 1 and 2 illustrate exemplary flowcharts of a training section including a plurality of training segments (denoted "sections" or "days") with educational contents and/or practical exercises (without distinction denoted "exercises") appearing on a screen of a user's terminal, where the user communication unit is running. Contents and exercises are provided by one or more tool boxes. Each day is concluded by a round of questions and answers ("Q&A"). If questions are answered incorrect ("NO"), the training segment ("day") has to be repeated the next time. Otherwise, the user can proceed to the next training segment ("day"). An optional HCP unit ("HCP APP") is communicatively linked to the user communication unit. At the end of the training section (day 3 in Fig. 1), the user receives feedback based on his/her performance. In Fig. 1, the feedback is transmitted from the HCP unit to the user communication unit.

The following examples provides two exemplary surveys including educational contents and practical exercises (in the following without distinction primarily referred to as patient education and counseling) appearing on a screen of a user terminal (herein referred to as Home Screen) on which the respective application (herein referred to as App SW/Program) is run. In these examples, each one of the contents and exercises is assigned to a consecutive day number reflecting in which order the contents and exercises are to be completed. This order is preferred. Nevertheless, the order may in some embodiments be different. Moreover, each content and exercise described in the following reflect individual embodiments of the invention. This means, the present invention encompasses any journey that combines 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, or 40 of the following contents and exercises ("days").

Each day is self-contained and shall be completed by the patient on the day it is assigned. Incomplete days (meaning that the user has not completed the respective content or exercise) will appear again on the next day on the Home Screen of the App SW. Each day a different aspect of tinnitus will be covered. Each daily section (referred to above as segment) should take about 15 minutes to complete, some a bit more. Exercises and assessments are integrated in the daily sections. From time to time, there will be speakers that teach and explain concepts. Each day may start with a brief summary of the previous content.

The following example contains educational and counseling content:

Day 1 - What is Tinnitus?
In the Education & Counselling Module of the Program important information about tinnitus is provided. Often, incorrect information about tinnitus can significantly influence the way one feels or acts. The user may be guided to a sound finder module that assists the user in self-determining his/her particular tinnitus tone.

Day 2 - Tinnitus Perception
In the last section it was learned that tinnitus sounds can be very different. And the Sound Finder was used to describe how the user perceives his/her tinnitus. The sound of tinnitus can be different from person to person. But also, other aspects of tinnitus perception are individual for each person and will provide important information about the tinnitus. This is the topic of Day 2. The section is concluded by a questionnaire based on the taught content.

Day 3 - What causes Tinnitus?
On day 3, it is focused on hearing loss, which often can be a cause of tinnitus. The hearing loss can be managed by using a hearing aid, which often also improves the tinnitus. Optionally, a speaker teaches and explains concepts. The section is concluded by a questionnaire based on the taught content.

Day 4 - Why do some people complain about tinnitus and others do not?

In the last session, it was taught that hearing loss could be a cause for tinnitus. That's because the brain can increase the "Central Auditory Gain" to compensate for reduced or missing input, but also might increase tinnitus sensation. On day 4, it is taught that tinnitus has very different effects on the well-being of people. Optionally, a speaker teaches and explains concepts. The section is concluded by a questionnaire based on the taught content.

Day 5 - Somatic Factors

Last time it was taught that emotional factors could have strong influence on the tinnitus. On day 5, it is taught that at your head, neck and muscular factors can influence tinnitus, so-called "Somatic Factors". Optionally, a speaker teaches and explains concepts. The section is concluded by a questionnaire based on the taught content.

Day 6 - Common Misunderstandings Part 1

On Day 6, common misunderstandings about Tinnitus are discussed. This section is structured like a quiz, meaning that each topic will start with a question, the answer will follow.

Day 7 - Common Misunderstandings Part 2

On Day 7 it is continued with the common misunderstandings about tinnitus in the form of a quiz.

Day 8 - Hearing Loss and Hearing Aids

Since hearing loss contributes to the impression of tinnitus in many people, it is worthwhile to spend few more minutes on this topic. Thus, on day 8, it is taught how hearing aids can support a sufferer, and how a hearing aid could have beneficial influences on the tinnitus. Optionally, a speaker teaches and explains concepts. The section is concluded by a questionnaire based on the taught content.

Day 9 - Managing Tinnitus

Hearing aids are a great tool to compensate hearing loss and have positive effects on tinnitus and well-being. On day 9, several other methods that can help managing tinnitus are taught. Optionally, a speaker teaches and explains concepts. The section is concluded by a questionnaire based on the taught content.

Day 10 - Relaxation

Last time habituation was introduced, a process whereby tinnitus sounds become filtered from their own conscious awareness resulting in perceived reduced tinnitus intensity. On day 10, relaxation and a relaxation exercise is presented. This may help to take the focus away from tinnitus. This exercise can be very helpful, and it is recommended that this exercise is integrated into the daily routine, at least 2-3 times per week. The section may start with the so called Five Well-Being Index from the World Health Organization (WHO-5). The answers can be related to the user's general well-being and quality of life.

Day 11 - Ear Wax Removal

Last time the relaxation exercise was introduced to reduce stress level. On day 11, very practical problem, ear wax and its removal, are discussed. An ear canal free of debris can improve hearing and can have a positive impact on tinnitus. Therefore, this topic is important. Optionally, a speaker teaches and explains concepts. The section is concluded by a questionnaire based on the taught content.

Day 12 - Hyperacusis and Tinnitus

On day 12, on a problem, where increased sensitivity to sound could start to cause problems, called "Hyperacusis" is focused. Optionally, a speaker teaches and explains concepts. The section is concluded by a questionnaire based on the taught content.

Day 13 - Protecting Hearing

On day 12, it was taught that some people have a higher or abnormal sensitivity with respect to everyday sounds, called Hyperacusis. In such a case it is not recommended to protect the hearing from normal environmental sounds (that are not concerningly loud), since this could enhance the discomfort for sounds even more. On day 13, it is taught under which circumstances a protection of hearing is important. Optionally, a speaker teaches and explains concepts. The section is concluded by a questionnaire based on the taught content.

Day 14 - Monitoring Tinnitus

On day 14, options to monitor tinnitus by using a diary is taught. The session may be short. Thus, it is recommended to perform the relaxation exercise as well. Optionally, a speaker teaches and explains concepts. The section is concluded by a questionnaire based on the taught content.

Day 15 - Use of Sound
On day 15, it is discussed if sound can be used to reduce tinnitus awareness. The section is concluded by a questionnaire based on the taught content.

Day 16 - Dealing with Sleep Problems
In the last session it was taught that sound can be used to reduce tinnitus awareness. In particular, when trying to fall in sleep, such sounds can be very helpful. On day 16, it is taught how to improve sleep. Optionally, a speaker teaches and explains concepts. The section is concluded by a questionnaire based on the taught content.

Day 17 - Dealing with Emotional Consequences of Tinnitus
On day 17, the last session of the Education & Counseling part of the tinnitus journey of this example, the topic relates to dealing with emotional consequences of tinnitus. Optionally, a speaker teaches and explains concepts. The section is concluded by a questionnaire based on the taught content.

Another exemplary journey includes mainly content from cognitive behavioral therapy and contains the following topics and exercises:

Day 1 - Course Intro
Day 2 - Relaxation Training Introduction
Day 3 - First Relaxation Training
Day 4 - Repeat First Relaxation Training
Day 5 - Second Relaxation Training
Day 6 - Repeat Second Relaxation Training and Sleep Exercise
Day 7 - Tinnitus Symbolization
Day 8 - Imagination Exercise
Day 9 - Repeat First Relaxation Training
Day 10 - Directing Attention
Day 11 - Repeat Relaxation Training
Day 12 - Recap on Directing Attention and Use of your Senses
Day 13 - Repeat Imagination Exercise
Day 14 - Short Relaxation Training
Day 15 - Repeat Short Relaxation Training and List of Senses
Day 16 - Stressful Situations and ABC Model
Day 17 - Repeat First Relaxation Training
Day 18 - ABC Model
Day 19 - Repeat Short Relaxation Training and ABC Model
Day 20 - Further Examples
Day 21 - Repeat Second Relaxation Training
Day 22 - Vicious Spiral
Day 23- Questioning Introduction
Day 24 - Questioning Examples
Day 25 - Repeat Short Relaxation Training Questioning Technique
Day 26 - Strategies against Error Traps 1
Day 27 - Repeat Thinking Traps, Short Relaxation Training & Sleep Exercise
Day 28 - Strategies against Error Traps 2
Day 29 - Repeat Counter Strategies & Relaxation Training
Day 30 - Responsibility Trap
Day 31 - Repeat Second Relaxation Training
Day 32 - Miracle Question
Day 33 - Repeat Thinking Traps, Counter Strategies & Sleep Exercise
Day 34 of CBT - Climb up the Solution Staircase
Day 35 - Repeat Solution Staircase & Short Relaxation Training
Day 36 - Coping Strategies
Day 37 - Human Health Model
Day 38 - Tips for Enjoyment
Day 39 - Repeat First Relaxation Training & Sleep Exercise

Day 40 - Self-Management and Prophylaxis

**Claims**

1. A tinnitus management system, the system comprising:

   a survey developing unit for developing and transmitting a survey to a user communication unit, the survey including a plurality of training sections, each training section including a plurality of training segments to be completed within a respective target timeframe, the training segments including educational contents and/or practical exercises;
   a progress determining unit for receiving and evaluating a user response received from the user communication unit, and repeatedly determining the number of completed training segments and/or training sections,
   a performance evaluation unit for comparing the target timeframe received from the survey developing unit with the actual duration needed for completing the respective training section and for determining a performance indicator indicative for the relative speed of completing the respective training section, a feedback unit for selecting one of a plurality of feedbacks based on the performance indicator received from the performance evaluation unit and for transmitting, after each training section, the feedback to the user communication unit, wherein the survey developing unit develops and/or adapts, after a training section, the subsequent training section or the following part of the survey based on the performance indicator determined for the respective training section.

2. The system of claim 1, wherein the system further comprises a plurality of user communication units, each one associated with a specific user and implemented on a terminal of a respective user.

3. The system of claim 1 or 2, wherein the system further comprises a health care professional (HCP) unit for tracking progress of the user and/or for tracking performance of the user and/or for communicating with the user.

4. The system of any of claims 1 to 3, wherein the HCP unit receives the feedback for confirmation or modification thereof before it is transmitted to the user communication unit.

5. The system of any of claims 1 to 4, wherein the target timeframe is defined so that on average one training segment is to be completed in 1 to 3 days.

6. The system of any of claims 1 to 5, wherein access of the user to a training segment is disabled, until the progress determining unit determines that each previous training segment has been completed.

7. The system of any of claims 1 to 6, wherein access of the user communication unit to an educational content is disabled, if the progress determining unit determines that a previous educational content has already been completed on the same day.

8. The system of any of claims 1 to 7, wherein the training segments are designed to be completed within 5 to 60 minutes.

9. The system of any of claims 1 to 8, wherein the system further comprises a wellbeing evaluation unit for determining a wellbeing score based on the user's response to a THI, GAD-7 and/or PHQ-9 questionnaire.

10. The system of claim 9, wherein the wellbeing evaluation unit determines a first wellbeing score and a second wellbeing score at a more advanced progress, and wherein the wellbeing evaluation unit compares the second wellbeing score with the first wellbeing score to determine a wellbeing development score, wherein the wellbeing development score indicates whether and/or how much the patient's wellbeing improved, stagnated or worsened.

11. The system of claim 10, wherein, if the wellbeing development score indicates that the patient's wellbeing improved, the survey development unit includes at least one additional training section in the survey and/or wherein, if the wellbeing development score indicates that the patient's wellbeing stagnated or worsened, the survey is discontinued.

12. The system of any of claims 1 to 11, wherein the survey development unit applies a dynamic, self-optimizing algorithm when including the additional training section based on the wellbeing development score and/or wherein the survey development unit applies a dynamic, self-optimizing algorithm when developing and/or adapting the subsequent training section or the following part of the survey based on the performance indicator.

13. A method of managing tinnitus, whereby:

a survey developing unit develops and transmits a survey to a user communication unit, the survey including a plurality of training sections, each training section including a plurality of training segments to be completed within a respective target timeframe, the training segments including educational elements and/or practical exercises;

a progress determining unit receives and evaluates a user response received from the user communication unit, and repeatedly determines the number of completed training segments and/or training sections,

a performance evaluation unit compares the target timeframe received from the survey developing unit with the actual duration needed for completing the respective training section and determines a performance indicator indicative for the relative speed of completing the respective training section,

a feedback unit selects one of a plurality of feedbacks based on the performance indicator received from the performance evaluation unit and transmits the feedback to the user communication unit, and

the survey developing unit develops and/or adapts subsequent training section(s) or the following part of the survey based on the performance indicator.

14. Use of a system as defined in any of claims 1 to 12 for managing tinnitus.

15. The use of claim 14 or the method of claim 13, wherein the managing of tinnitus is based on assisting a user in perceiving the tinnitus less burdensome.

Fig. 1

Tool box

```
┌─────────────────────┐
│ Previous Sections / │
│       Days          │
└─────────────────────┘
          │
          ▼
┌─────────────────────┐────────────────┐                    ┌──────────────┐
│  Section N / Day M  │                │                    │     HCP      │
├─────────────────────┤     ┌──────────┐                    │   APP via    │
│     Section N       │────▶│   Tool   │                    │    Server    │
│  Exercise X & Z M   │     │   box    │                    └──────────────┘
└─────────────────────┘     └──────────┘                            ▲
          │                                                          │
   NO     ▼                                                          │
      ╱ Day M + Q&A ╲──────────────────────────────────────────────┤
      ╲  complete   ╱                                               │
          │                                                          │
   YES    ▼                                                          ▼
┌─────────────────────┐          ┌──────────────────────────────────────┐
│   Feedback Screen   │◀─────────│      Analyse for Situations          │
│                     │          │      (SIT) and Conditions            │
└─────────────────────┘          │             (CON)                    │
                                 └──────────────────────────────────────┘
```

Fig. 2

14

Europäisches
Patentamt

European
Patent Office

Office européen
des brevets

# EUROPEAN SEARCH REPORT

Application Number

EP 21 16 6235

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | CN 111 477 299 A (GUANGZHOU AIBORUN MEDICAL TECH CO LTD) 31 July 2020 (2020-07-31) * paragraphs [0007], [0061], [0071] - [0075] * | 1-15 | INV. G16H20/70 |
| X | CN 111 420 213 A (EYE & ENT HOSPITAL OF FUDAN UNIV) 17 July 2020 (2020-07-17) * abstract * | 1-15 | |
| X | WO 2021/032327 A1 (AURELIYM GMBH [DE]) 25 February 2021 (2021-02-25) * page 15, line 19 - page 27, line 3 * | 1-15 | |
| A | US 2019/074081 A1 (EASTON CAROLINE J [US] ET AL) 7 March 2019 (2019-03-07) * paragraphs [0017] - [0110] * | 1-15 | |

TECHNICAL FIELDS
SEARCHED      (IPC)

G16H

The present search report has been drawn up for all claims

| Place of search | Date of completion of the search | Examiner |
|---|---|---|
| Munich | 14 September 2021 | Díaz de Lezana, C |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 21 16 6235

This annex lists the patent family members relating to the patent documents cited in the above-mentioned European search report.
The members are as contained in the European Patent Office EDP file on
The European Patent Office is in no way liable for these particulars which are merely given for the purpose of information.

14-09-2021

| Patent document cited in search report | | Publication date | Patent family member(s) | Publication date |
|---|---|---|---|---|
| CN 111477299 | A | 31-07-2020 | NONE | |
| CN 111420213 | A | 17-07-2020 | NONE | |
| WO 2021032327 | A1 | 25-02-2021 | NONE | |
| US 2019074081 | A1 | 07-03-2019 | NONE | |

EPO FORM P0459

For more details about this annex : see Official Journal of the European Patent Office, No. 12/82